# EUROPEAN PATENT APPLICATION

(11) **EP 1 068 853 A1**
(43) Date of publication of application: **17.01.2001**
(21) Application number: 99902915.0
(22) Date of filing: 16.02.1999
(51) Int. Cl.: A61K 7/00, A61K 7/06

(54) **COVERING SHEET FOR SKIN AND HAIR**

(30) Priority: 30.03.1998 JP 8358098; 09.06.1998 JP 16022798
(71) Applicant: Kao Corporation, Tokyo 103-8210 (JP)
(72) Inventor: SATO, Nobuya, Kao Corp. Res Laboratories, Tochigi 321-3497 (JP); YAMAUCHI, Michihide, Kao Corp. Res. Laboratories, Haga-gun, Tochigi 321-3497 (JP)
(74) Representative: Marsh, Roy David
(86) International application number: JP9900660
(87) International publication number: WO9949833

(57) **Abstract**

The present invention is directed to a sheet for covering the skin or hair containing 100 parts by weight of a thermoplastic resin (A) and 0.01-200 parts by weight of a medical ingredient (B) acting on the skin or hair, and to a method for supplying the ingredient (B) to the skin or hair topically by use of the sheet.

The sheet causes a useful medical ingredient to act effectively on a site of the skin or hair to which the sheet is applied, while providing a comfortable sensation during use.

## Description

### Technical Field

The present invention relates to a sheet for covering the skin or hair, and more particularly to a sheet for covering the skin or hair which is capable of causing a useful medical ingredient to act effectively on the skin or hair while providing a comfortable sensation during use.

### Background Art

In general, when any of variety of medical ingredients is applied to, for example, the skin or hair, such ingredient is directly applied thereto, or alternatively, paste containing such ingredients is applied in advance to a fabric substrate such as a non-woven fabric, and the paste-covered fabric is then applied to the skin or hair. For example, Japanese Patent Application Laid-Open (*kokai*) No. 62-286908 discloses a skin cosmetic agent for external use which comprises castor oil and beeswax formed into a jelly-like product. However, problems may arise with this presentation of the product; it is difficult to use, in that it may stain the clothing during use or remain on the skin after use. Japanese Patent Application Laid-Open (*kokai*) No. 55-92306 discloses a pack agent comprising an adhesive ingredient incorporating a cosmetic ingredient, the intended manner of use being direct application to the skin. Similarly, Japanese Patent Application Laid-Open (*kokai*) Nos. 7-258060 and 9-295929 disclose a pack agent comprising an adhesive ingredient incorporating a whitening agent, and a pack agent comprising an adhesive ingredient incorporating an anti-wrinkle agent, respectively, which packs are assumed to be affixed to the skin. Japanese Patent Application Laid-Open (*kokai*) No. 2-36112 discloses a covering film for packing in which a heat-softened film is applied to the face or a part of the body, and then the film is cooled and hardened. In addition, Japanese Patent Application No. 9-194342 discloses an agent to prevent roughening of the skin comprising a polymer exhibiting high adhesion to skin. However, in the inventions disclosed in the above publications, a film (or sheet) must be affixed to the skin, and thus the film (or sheet) contains large amounts of a tacky ingredient. Therefore, such a film (or sheet) adheres firmly to the skin, and may induce an uncomfortable sensation or may cause problems when peeled off. In addition, when such a film (or sheet) is peeled off the skin, portions of the film (or sheet) may remain on the skin because of low film (or sheet) strength.

In view of the foregoing, an object of the present invention is to provide a novel composition for external use, which raises no problems associated with adhesion thereof to the skin and which can effectively supply a predetermined medical ingredient to a desired skin site and facilitate action of the ingredient on the site.

### Disclosure of the Invention

Conventionally, in order to cause a medical ingredient to act effectively on the skin, an adhesive layer is formed on a sheet bearing the ingredient, and the sheet is affixed to the skin. However, quite surprisingly, the present inventors have found that a thermoplastic resin sheet containing merely a medical ingredient in a predetermined amount is capable of causing the ingredient to act effectively on the skin or similar portions of the body through the effect of moistening the skin, etc. (hereinafter the effect will be referred to simply as "moistening effect") provided by the sheet, and that the problems occurring when the sheet is peeled off can be eliminated.

Accordingly, the present invention provides a sheet for covering the skin or hair comprising 100 parts by weight of a thermoplastic resin (A) and 0.01 to 200 parts by weight of a medical ingredient (B) acting on the skin or hair.

The present invention also provides a method for supplying the ingredient (B) to the skin or hair topically, characterized by covering the skin or hair with the above-described sheet.

### Best Mode for Carrying Out the Invention

As used herein, the term "sheet" broadly refers to any material which can cover a site to which the material is applied along the curved surface of the site. For example, such materials encompass films having a thickness of 100 µm or less, and sheet-like materials, including fiber-containing products such as non-woven fabric.

The thermoplastic resin (A) employed in the sheet of the present invention may be a commonly used thermoplastic resin. Examples of such resins include polyolefin resins such as polyethylene (e.g., low-density polyethylene, high-density polyethylene, linear low-density polyethylene, or very-low-density polyethylene), polypropylene, polybutene, and poly-4-methylpentene-1; polyolefin-modified resins or copolymers such as an ethylene-vinyl acetate copolymer, an ethylene-vinyl alcohol copolymer, an ethylene-methyl methacrylate copolymer, and an ethylene-α-olefin copolymer; polyamides such as 6-Nylon and 66-Nylon; polyesters such as polyethylene terephthalate and polybutylene terephthalate; polyvinyl chloride; polyvinylidene chloride and modified resins or copolymers thereof; polystyrene; polyvinyl acetate; polyacrylonitrile; polycarbonate; and polyacrylate.

In order to impart flexibility to a sheet and ensure that its properties allow it to conform itself to the skin or hair, it is preferable to employ an ethylene-α-olefin copolymer having a density of less than 0.920 g/cm³ (e.g., a copolymer produced by use of a cyclopentadienyl catalyst); polypropylene having a flexural elastic modulus of 5,000 cN/cm² or less (as measured in accordance with ASTM-D790); and a polymer which is mixed with ethylene-propylene rubber during polymerization (e.g., Catalloy, product of Montell).

In addition, a polymer known as thermoplastic elastomer (TPE) may be employed in the present invention. Examples of TPEs include styrene TPE comprising polystyrene serving as a hard segment, and polybutadiene, polyisoprene, or poethylene-polybutylene serving as a soft segment; olefin TPE comprising polypropylene serving as a hard segment and EPDM or EPM serving as a soft segment; urethane TPE comprising polyurethane serving as a hard segment and polyether or polyester serving as a soft segment; ester TPE comprising polyester serving as a hard segment and polyether or polyester serving as a soft segment; PVC-TPE; butyl rubber graft polyethylene comprising polyethylene and butyl rubber; 1,2-polybutadiene comprising 1,2-syndiotactic polybutadiene and amorphous polybutadiene; trans-1,4-polyisoprene comprising trans-1,4-polyisoprene and amorphous polyisoprene; an ionomer comprising metal carboxylate cluster and amorphous polyethylene; and natural rubber TPE comprising polypropylene and natural rubber.

When such a thermoplastic elastomer is incorporated into the sheet, the sheet acquires flexibility and has those properties which allow it to conform closely to the skin or hair. In order to obtain such effects, a thermoplastic elastomer is incorporated, into 100 parts by weight of the resin (A), in an amount of 1-95 parts by weight, preferably 10-80 parts by weight, more preferably 20-60 parts by weight.

Examples of the medical ingredient (B) which may be employed in the present invention include a moisturizer, a whitening agent, a UV absorber, a slimming agent, a circulation promoter, an astringent, an anti-inflammatory agent, a wrinkle-formation preventive and ameliorating agent, a cooling agent, a warming agent, a hair remover, a hair growing agent, a hair-growth regulating agent, and a hair nourishing agent.

### Specific examples are:

(1) natural fats and oils, and hydrogenated oils or glyceride derivatives which are obtained through hydrogenation of the natural fats and oils, such as beef tallow, milk fat, lard, sardine oil, mackerel oil, tuna oil, shark liver oil, linseed oil, safflower oil, sunflower oil, soybean oil, corn oil, peanut oil, sesame oil, rapeseed oil, olive oil, palm oil, palm-kernel oil, coconut oil, and castor oil;
(2) middle and higher fatty acids, such as butyric acid, caproic acid, caprylic acid, capric acid, lauric acid, myristic acid, palmitic acid, palmitoleic acid, stearic acid, oleic acid, linoleic acid, linolenic acid, moroctic acid, arachidic acid, eicosenoic acid, arachidonic acid, eicosapentaenoic acid, behenic acid, erucic acid, docosapentaenoic acid, docosahexaenoic acid, lignoceric acid, selacholeic acid, and isostearic acid;
(3) ester derivatives formed of the aforementioned higher fatty acids (2) and glycerin or polyglycerins, such as monoesters and diesters;
(4) higher alcohols which are obtained through reduction of the aforementioned higher fatty acids (2), such as lauryl alcohol, cetyl alcohol, stearyl alcohol, and oleyl alcohol, and ester derivatives of the alcohols;
(5) hydrocarbons such as liquid paraffin, paraffin, vaseline, paraffin micro-crystalline wax, ceresine, pristane, and squalane;
(6) waxes such as carnauba wax, beeswax, and lanolin;
(7) ceramides and analogue substances thereof, cholesteryl esters, vitamins (e.g., vitamin A, vitamin B, vitamin C, vitamin D, vitamin E, and derivatives of the vitamins), polyoxypropylene fatty acid esters, and cholesterols;
(8) crude drugs, Chinese herbal drugs, herbs, perfumes, and silicones;
(9) UV screen, UV absorption, or UV protection agents (e.g., benzophenone compounds, p-aminobenzoic acid compounds, methoxycinnamic acid compounds, and salicylic acids); titanium oxide, zirconium oxide, iron oxide, and micro particle treatment substances thereof; and surface treatment or composite treatment substances of silicone or zirconia·alumina.

One or more substances of these may be employed in the present invention. Of these medical ingredients (B), oily substances of (1) through (6) are preferably employed as the medical ingredient (B). If necessary, substances of (1) through (6), and (7) or weak alkaline inorganic compounds such as magnesium oxide and calcium hydroxide may further be incorporated into the sheet in appropriate amounts.

The amount of medical ingredient (B) is 0.01-200 parts by weight, preferably 0.1-100 parts by weight, more preferably 1-50 parts by weight, on the basis of 100 parts by weight of the resin (A). When the amount of the ingredient (B) is less than 0.01 parts by weight, the sheet does not exhibit the desired properties, whereas when the amount is in excess of 200 parts by weight, stable high-yield production of the sheet cannot be achieved.

In the sheet of the present invention, medical ingredient (B) is dispersed in resin (A) so that a required amount of the ingredient (B) can be supplied to the covered site when a target site is covered with the sheet. Thus, the sheet does not require an adhesive layer that is essential to conventional patches.

The sheet of the present invention can be produced through, for example, the following process.

Firstly, the resin (A) is mixed with the medical ingredient (B). The resin (A) may be heated and melted, and then mixed with the ingredient (B). Alternatively, the resin (A) may be dissolved in a predetermined solvent, and then mixed with the ingredient (B).

In production, the sheet may be shaped by use of a T die, an inflation apparatus, or a calender. The sheet may be produced by cutting a film or sheet comprising a single layer or two or more layers into a predetermined size or shape. The resin (A) and the medical ingredient (B) may be kneaded at a temperature at which the resin (A) is melted or a higher temperature through a non-woven fabric production process, such as a spun bond process or melt blowing, to thereby produce a non-woven fabric.

The sheet may be shaped after the resin (A) is mixed with the medical ingredient (B) at high pressure by use of a solvent which is compatible with the resin (A) at high pressure and which is separated from the resin at low pressure. The mixing process is usually employed to produce flash spinning fiber. The sheet produced through the process takes the form of a fibrous aggregation, and is gas-permeable and waterproof

The sheet of the present invention which is produced through the above process may be easily applied to a portion of the body by winding a bandage or a similar material around the site.

When the surface of the sheet of the present invention is made flat and smooth, the sheet *per se* of the present invention exhibits a strong adhesive force between sheets. Therefore, the sheet does not stick to the skin or clothing, the body is tightly wrapped and sealed with the sheet, the evaporation of moisture from the skin surface can be suppressed, and even when there is only a small amount of the medical ingredient (B) contained in the sheet , it can be effectively absorbed percutaneously through the moistening effect.

When the sheet is applied as described above, the cohesive force between the sheets is 2,000 cN/4 cm² or more, preferably 3,000 cN/4 cm² or more, more preferably 5,000 cN/4 cm² or more. The cohesive force between the sheets is obtained as follows: two sheets having a length of 75 mm and a width of 20 mm are made to overlap each other at 20°C such that the area of the overlapped region is 4 cm²; a load was applied onto the overlapped portion under a rubber roller of 400 g (10 cm/sec; 2 reciprocations); and a shear peel strength of the sheets is obtained by use of Tensilon (product of Orientec).

In order to cause the sheet to have a cohesive force which is equal to or higher than that of the resin (A), an oily ingredient may be incorporated into the sheet as the medical ingredient (B) or another ingredient. Varying the type or amount of the oily ingredient can control the cohesive force.

For example, when natural fats and oils, hydrogenated oils or glyceride derivatives which are obtained through hydrogenation of the natural fats and oils, higher fatty acids, higher alcohols or ester derivatives thereof, ester derivatives formed of higher fatty acids and glycerin or polyglycerins, such as monoesters and diesters, or hydrocarbons are added to the resin (A), the resin is softened and a strong cohesive force is generated between the sheets. The thus-generated cohesive force brings the sheets to tightly contact to each other, without permitting the sheets to stick to or adhere to the skin or clothing. The cohesive force between the sheets may be increased through reduction in the density of the resin, in addition to the use of the aforementioned oily ingredient.

When the sheet is applied to an uneven part of the body or where the body flexes, the sheet may be held in place by means of any fixation method, in addition to the adhesion force of the sheet. For example, supporters, gloves, socks, and stockings may be employed to thereby bring the sheet into effective contact with the intended site of the body.

When the sheet is employed in such a manner, the sheet preferably fits closely and flexibly against the skin. In view of the foregoing, the sheet of the present invention preferably has a thickness of 5-200 µm, more preferably 5-100 µm.

When the sheet of the present invention is in the form of non-woven fabric, the flexibility of the sheet may be represented better by basis weight than thickness. The basis weight of the sheet is preferably 5-200 g/m², more preferably 5-100 g/m². When the sheet has a basis weight falling within such ranges, body-contour conformity (tight contact) of the sheet to the body is enhanced, and the flexibility thereof is improved. When another non-woven fabric or sheet is laminated onto the sheet of the present invention or when a composite sheet is formed of the sheet of the present invention, the basis weight of the laminated sheet or the composite sheet is the sum of the basis weight of the sheet of the present invention and that of the additional sheet(s). In this case, the sum of the basis weights may exceed the above optimal range given for the basis weight of the sheet of the present invention.

In order to enhance flexibility of the sheet produced from resin (A), it is preferable that the aforementioned thermoplastic elastomer, oily ingredient, or flexible polyolefin resin be added to the sheet. When flexibility of the sheet is represented by percentage of stretching, the sheet of the present invention is capable of being stretched by 50% or more. In order to obtain excellent conformity of the sheet to the body, the modulus when the sheet is stretched by 50% is 1-2,000 cN/10 mm, preferably 10-1,000 cN/10 mm, and more preferably 10-500 cN/10 mm.

Because the sheet of the present invention is used to cover the skin or hair, it is better if the sheet does not adhere to the skin or hair to protect the skin or hair from any damage. As used herein, the phrase "the sheet does not adhere to the skin" refers to the case in which the sheet substantially does not adhere to the skin, i.e., peeling off the sheet covering the skin does not require additional force. However, when the sheet is applied to a portion of the body having an uneven contour or to only a part of the body, a tackifier may be incorporated into the sheet so long as the intended effect of the present invention is not impeded. Examples of tackifiers which may be employed in the present invention include natural resins such as rosin and dammar, polyterpene resins, and aliphatic hydrocarbon resins. These tackifiers are described in "Kobunshi Kako, special issue 8, Nenchaku" (republished by Kobunshi Kankokai, on July 15, 1976, page 105, Table 1). Of these tackifiers, one or more species may be employed in the sheet of the present invention. The amount of a tackifier which is incorporated into the sheet must be determined such that the tackifier does not cause any damage to the skin. In view of the foregoing, the amount of a tackifier which is incorporated into the polyolefin resin (A) (100 parts by weight) is 99 parts by weight or less, preferably 75 parts by weight or less, more preferably 50 parts by weight or less.

The sheet of the present invention comprises the medical ingredient (B) on the inside and on the surface of the sheet prepared from resin (A). Therefore, in order to prevent the ingredient (B) which is present on the sheet surface and opposed against the skin from adhering to another part of the body or to the clothing, any barrier material may be laminated onto the sheet so as to form a multi-layer structure. For example, a thermoplastic resin or a thin film made from an inorganic compound not containing the ingredient (B) may be employed as a back-coating, or a non-woven fabric may be laminated onto the sheet. When another non-woven fabric or sheet is laminated onto the sheet of the resin (A) or a composite sheet is formed of the sheet of the resin (A), the overall thickness of the laminated sheet or the composite sheet may exceed the above preferable range for the thickness of the sheet of the present invention.

The sheet of the present invention may be embossed during production in order to control the sensation produced on the skin. By whatever means the sheet is produced to increase its bulkiness, the effect of the present invention is not impeded so long as the thickness of the sheet basically falls within 5-200 µm as described above.

The sheet of the present invention can be produced in such a way that it possesses an appropriate moisture permeability to impart both a moistening sensation and a refreshing sensation to the skin. Specifically, the moisture permeability of the sheet may be controlled by adding any of the following polymers to the sheet: ethylene copolymers having a polar group, such as an ethylene-vinyl acetate copolymer of relatively low moisture permeability; and polyurethane elastomers or polyester elastomers which may have high moisture permeability. These polymers may be employed singly or in combination. Incidentally, micropores may be formed in the sheet. No particular limitation is imposed on the method for creating micropores in the sheet, and micropores are formed by means of, for example, laser processing or discharge processing as disclosed in Japanese Patent Application Laid-Open (*kokai*) No. 4-279321. Alternatively, inorganic filler can be added to the sheet and the pores are then formed through stretching. A different method again is where pores can be formed through melting using heated pins.

The aforementioned sheet (non-woven fabric) produced through flash spinning, melt blowing, or spun-bond processing is permeable to the air. In order to control the air permeability of such a sheet, the sheet may be treated by pressing it with a roller.

### Examples

### Example 1

Castor oil serving as a medical ingredient (B) (5 parts by weight) was added to linear low-density polyethylene (Ultzex 15100, product of Mitsui Chemicals, Inc.) (80 parts by weight) and very low density polyethylene (DFDB9042, product of Nippon Unicar Co., Ltd.) (20 parts by weight), serving as a resin (A). The resultant mixture was melted at 170°C and kneaded, and then shaped at 200°C by use of a T die, to thereby produce the sheet of the present invention having a thickness of 30 µm.

### Examples 2 through 15

The sheets of the present invention were produced in a manner similar to that of Example 1 under conditions shown in Tables 1 through 4.

### Comparative Example 1

A sheet having a thickness of 20 µm was produced by use of linear low-density polyethylene (Ultzex 15100, product of Mitsui Chemicals, Inc.) serving as a resin (A).

### Test Example 1

The five persons enrolled in the test were covered or patched with the sheets of Examples 1 through 15 and Comparative Example 1 on the backs of their hands, heels, knees, and necks for 30 minutes. The sensations caused by the sheets (tight contact, conformity, moistness after use, and peelability) were organoleptically evaluated in accordance with the below-described criteria. The results are shown in Tables 1 through 4.

### 〈Evaluation criteria〉

(1) Cohesive force
   Cohesive force between the sheets were measured as described above.
(2) Conformity
   Conformity was determined by the number of persons who reported "the sheet did not slip off the site to which the sheet had been applied, and the sheet conformed to movement of the joint."
   Four or more: good
   Three or less: poor
(3) Moistness after use
   Moistness after use was determined by the number of persons who reported "the site to which the sheet had been applied became moistened after use, or a reduction in the number of wrinkles was observed."
   Four or more: good
   Three or less: poor
(4) Peelability
   Peelability was determined by the number of persons who reported "there was no irritation to the skin, and no debris of epidermis or hair was observed on the surface of the sheet after use."
   Four or more: good
   Three or less: poor

**Table 1**

| (unit: parts by weight) | | | | | | | |
|---|---|---|---|---|---|---|---|
| Resin/Medical ingredient/Production conditions/Properties | Product name | Maker | Examples | | | | |
| | | | 1 | 2 | 3 | 4 | 5 |
| Low-density polyethylene | Mirason 11P | #1 | | 20 | 10 | | |
| Linear low-density polyethylene | Ultzex 15100 | #1 | 80 | | | | |
| very low-density polyethylene | DFDB 9042 | #2 | 20 | | | | |
| Ethylene-α-olefin copolymer | Affinity EG8200 | #3 | | 80 | | | |
| Ethylene-vinyl acetate copolymer | Evaflex P2807 | #4 | | | 45 | | |
| Ethylene-methacrylate | Acryft CM4013 | #5 | | | 45 | | |
| Polypropylene | KS357P | #6 | | | | 80 | |
| Polypropylene | PF-814 | #6 | | | | 20 | |
| Polypropylene | F569D | #7 | | | | | 60 |
| Polybutene | Tafmer BL2481 | #1 | | | | | 40 |
| Castor oil (natural product) | | | 5 | | | | |
| Olive oil (natural product) | | | | 10 | | | |
| Soybean oil (natural product) | | | | | 30 | | |
| Corn oil (natural product) | | | | | | 30 | |
| Coconut oil (natural product) | | | | | | | 50 |
| Kneading temperature (°C) | | | 170 | 160 | 170 | 200 | 200 |
| Shaping method | | | T die | | | | Inflation |
| Shaping temperature (°C) | | | 200 | 200 | 200 | 220 | 220 |
| Sheet pressure (µm) | | | 30 | 10 | 20 | 25 | 25 |
| Cohesive force (cN/4cm²) | | | 6000 | 5000 | 5500 | 3000 | 3500 |
| Conformity | | | Good | Good | Good | Good | Good |
| Moistness after use | | | Good | Good | Good | Good | Good |
| Peelability | | | Good | Good | Good | Good | Good |

| | | | | | | | |
|---|---|---|---|---|---|---|---|
| #1 Mitsui Chemical, Inc., | | | | | | | |
| #2 Nippon Unicar Co., Ltd., | | | | | | | |
| #3 Dow Chemical Co., | | | | | | | |
| #4 Mitsui-Du Pont Co., | | | | | | | |
| #5 Sumitomo Chemical Co., Ltd., | | | | | | | |
| #6 Monter S.p.A, | | | | | | | |
| #7 Grand Polymer | | | | | | | |

**Table 2**

| (Incorporation unit: parts by weight) | | | | | | | |
|---|---|---|---|---|---|---|---|
| Resin/Medical ingredient/Conditions for production/Properties | Product name | Maker | Examples | | | | |
| | | | 6 | 7 | 8 | 9 | 10 |
| Low-density polyethylene | Mirason 11P | #1 | | | | 10 | 80 |
| Linear low-density polyethylene | Ultzex 15100 | #1 | 80 | | | | |
| Linear low-density polyethylene | Ultzex 2080 | #1 | | 80 | | 50 | |
| Very low-density polyethylene | DFDB9042 | #2 | | | | 40 | |
| Ethylene-α-olefin copolymer | Affinity EG8200 | #3 | | | | | 20 |
| Ethylene-α-olefin copolymer | Tafmer S4030 | #1 | | | 40 | | |
| Ethylene-ethyl acrylate-maleic anhydride copolymer | Bondine TX8030 | #5 | | 20 | | | |
| Polypropylene | F569D | #7 | | | 60 | | |
| Styrene elastomer | Kraton G1657 | #8 | 20 | | | | |
| Olive oil (natural product) | | | | | | | 0.1 |
| Corn oil (natural product) | | | 35 | 15 | | | |
| Rapeseed oil (natural product) | | | | | 25 | 15 | |
| Vitamin E (natural product) | | | | 0.1 | | | |
| Ceramide (synthetic product) | | | | | 1 | 1 | |
| Kneading temperature (°C) | | | 180 | 180 | 200 | 180 | 180 |
| Shaping method | | | T die | | | | |
| Shaping temperature (°C) | | | 220 | 200 | 220 | 200 | 200 |
| Sheet pressure (µm) | | | 20 | 25 | 75 | 25 | 200 |
| Cohesive force (cN/4cm²) | | | 6500 | 7000 | 4000 | 5000 | 3000 |
| Conformity | | | Good | Good | Good | Good | Good |
| Moistness after use | | | Good | Good | Good | Good | Good |
| Peelability | | | Good | Good | Good | Good | Good |

| | | | | | | | |
|---|---|---|---|---|---|---|---|
| #1 Mitsui Chemical, Inc., | | | | | | | |
| #2 Nippon Unicar Co., Ltd., | | | | | | | |
| #3 Dow Chemical Co., | | | | | | | |
| #5 Sumitomo Chemical Co., Ltd., | | | | | | | |
| #7 Grand Polymer, | | | | | | | |
| #8 Shell Kagaku K.K. | | | | | | | |

**Table 3**

| (Incorporation unit: parts by weight) | | | | | |
|---|---|---|---|---|---|
| Resin/Medical Ingredient/Conditions for production/Properties | Product name | Maker | | | |
| | | | 11 | 12 | 13 |
| Low-density polyethylene | Mirason 11P | #1 | | | |
| Linear low-density polyethylene | SP2520 | #1 | 60 | 60 | |
| Ethylene-α-olefin copolymer | Affinity EG8150 | #3 | 40 | 40 | |
| Ethylene-vinyl acetate copolymer | Evaflex P2807 | #4 | | | 100 |
| Urethane elastomer | Toyobo Urethane E3080AK | #5 | | | |
| Ester elastomer | Pelprene P-30B05 | #5 | | | |
| Olive oil (natural product) | | | 3 | 10 | |
| Soybean oil (natural product) | | | | | 30 |
| Rapeseed oil (natural product) | | | | | |
| Ceramide (synthetic product) | | | | | |
| Kneading temperature (°C) | | | 180 | 180 | 170 |
| Shaping method | | | T die | | |
| Shaping temperature (°C) | | | 200 | 200 | 200 |
| Sheet pressure (µm) | | | 100 | 35 | 20 |
| Cohesive force (cN/4cm²) | | | 8000 | 7500 | 7500 |
| Conformity | | | Good | Good | Good |
| Moistness after use | | | Good | Good | Good |
| Peelability | | | Good | Good | Good |

| | | | | | |
|---|---|---|---|---|---|
| #1 Mitsui Chemical, Inc., | | | | | |
| #3 Dow Chemical Co., | | | | | |
| #4 Mitsui-Du Pont Co., | | | | | |
| #5 Toyobo Co., Ltd. | | | | | |

**Table 4**

| (Incorporation unit: parts by weight) | | | | | |
|---|---|---|---|---|---|
| Resin/Medical ingredient/Conditions for production/Properties | Product name | Maker | Examples | | Comp. Ex. |
| | | | 14 | 15 | 1 |
| Low-density polyethylene | Mirason 11P | #1 | | | 100 |
| Linear low-density polyethylene | SP2520 | #1 | | | |
| Ethylene-α-olefin copolymer | Affinity EG8150 | #3 | | | |
| Ethylene-vinyl acetate copolymer | Evaflex P2807 | #4 | | | |
| Urethane elastomer | Toyobo Urethane E3080AK | #5 | 10 | | |
| Ester elastomer | Pelprene P-30B05 | #5 | | 100 | |
| Rapeseed oil (natural product) | | | 10 | 10 | |
| Ceramide (synthetic product) | | | 1 | 1 | |
| Kneading temperature (°C) | | | 180 | 180 | - |
| Shaping method | | T die | | | |
| Shaping temperature (°C) | | | 200 | 200 | 200 |
| Sheet pressure (µm) | | | 30 | 25 | 20 |
| Cohesive force (cN/4cm²) | | | 6000 | 6000 | 0 |
| Conformity | | | Good | Good | Poor |
| Moistness after use | | | Good | Good | Poor |
| Peelability | | | Good | Good | Good |

| | | | | | |
|---|---|---|---|---|---|
| #1 Mitsui Chemical, Inc., | | | | | |
| #3 Dow Chemical Co., | | | | | |
| #4 Mitsui-Du Pont Co., | | | | | |
| #5 Toyobo Co., Ltd. | | | | | |

As is apparent from Tables 1 through 4, the sheet of the present invention provides excellent tight contact and conformity to the skin, and also provides excellent moistness to the skin due to the effect of a medical ingredient contained in the sheet. In addition, the sheet causes no irritation and, upon peeling, little damage to the skin.

### Test Example 2

The arms of the two persons enrolled in the test were placed in an atmosphere (temperature: 25°C, humidity: 50%) for 30 minutes, and then the water content of the horny layers of the arms was measured in reference to the conductivity of skin surface. The conductivity was measured by use of a high-frequency resistance meter (model: SKICON-200, product of Hamamatsu). In a separate experiment, the procedure of Example 1 was repeated, except that olive oil (10 parts by weight) was employed as a medical ingredient, to thereby obtain a film. Subsequently, the right arm of each person enrolled in the test was covered with the thus-obtained film for 15 minutes, and then the film was removed from the arm. Fifteen minutes after removal of the film, the water content of the horny layers of the right arm was measured again. As a control, the left arm of each person was covered with the film of Comparative Example 1, and the water content of the horny layers of the arm was measured in a manner similar to that described above. The results are shown in Table 5.

**Table 5**

| | | Water content of horny layers (µS) | |
|---|---|---|---|
| | | Tested person A | Tested person B |
| Right Arm | Before covering | 5.2 | 8.3 |
| | After covering (Sheet of Present Invention) | 23.0 | 44.2 |
| Left Arm | Before covering | 7.2 | 8.7 |
| | After covering (Control) | 7.7 | 20.3 |

As is apparent from Table 5, the sheet of the present invention facilitates the medical ingredient contained in the sheet to exhibit its effect (in this case, the moistening effect of olive oil) through covering with the sheet.

### Industrial Applicability

The sheet of the present invention exhibits the following effects through wrapping, in addition to the above-described effects. Namely, the surface of the skin is sealed with the sheet through wrapping, and thus a medical ingredient contained in the sheet rapidly permeates the skin and the ingredient is effectively absorbed percutaneously through the moistening effect. In addition, the skin is protected from external irritations through wrapping with the sheet. For example, the skin can be prevented from chapping which is caused by scratching an itchy portion of the skin.

Moreover, since the sheet of the present invention has a simple structure, the sheet can be easily shaped and can be produced at an improved production efficiency.

## Claims

1. A sheet for covering the skin or hair comprising 100 parts by weight of a thermoplastic resin (A) and 0.01 to 200 parts by weight of a medical ingredient (B) acting on the skin or hair.

2. A sheet according to claim 1, which comprises no adhesive layer other than the sheet itself containing the ingredients (A) and (B).

3. A sheet according to claim 1 or 2, wherein the ingredient (A) is a polyolefin resin.

4. A sheet according to claim 1 or 2, wherein the ingredient (B) is selected from the group consisting of a moisturizer, a whitening agent, a UV absorber, a slimming agent, a circulation promoter, an astringent, an anti-inflammatory agent, a wrinkle-formation preventive and ameliorating agent, a cooling agent, a warming agent, a hair remover, a hair growing agent, a hair-growth regulating agent, and a hair nourishing agent.

5. A method for supplying a medical ingredient acting on the skin or hair (B) to the skin or hair topically, characterized by covering the skin or hair with a sheet comprising 100 parts by weight of a thermoplastic resin (A) and 0.01 to 200 parts by weight of the medical ingredient (B).
